(19)

Europäisches
Patentamt

European
Patent Office

Office européen
des brevets

(11)  **EP 3 154 407 B1**

(12)  # EUROPEAN PATENT SPECIFICATION

(45) Date of publication and mention
of the grant of the patent:
**02.06.2021 Bulletin 2021/22**

(21) Application number: **14730530.4**

(22) Date of filing: **16.06.2014**

(51) Int Cl.:
*A61B 3/00* (2006.01)          *A61B 3/113* (2006.01)
*G06K 9/00* (2006.01)

(86) International application number:
**PCT/EP2014/062604**

(87) International publication number:
**WO 2015/192879 (23.12.2015 Gazette 2015/51)**

(54)  **A GAZE ESTIMATION METHOD AND APPARATUS**

BLICKBEURTEILUNGSVERFAHREN UND -VORRICHTUNG

PROCÉDÉ ET APPAREIL D'ESTIMATION DE REGARD

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**19.04.2017 Bulletin 2017/16**

(73) Proprietor: **Fondation de L'institut de Recherche
Idiap
1920 Martigny (CH)**

(72) Inventors:
• **FUNES MORA, Kenneth Alberto
1007 Lausanne (CH)**
• **ODOBEZ, Jean-Marc
1815 Clarens (CH)**

(74) Representative: **P&TS SA (AG, Ltd.)
Avenue J.-J. Rousseau 4
P.O. Box 2848
2001 Neuchâtel (CH)**

(56) References cited:
**EP-A1- 2 521 007**

• **ARANTXA VILLANUEVA ET AL: "A geometric
approach to remote eye tracking", UNIVERSAL
ACCESS IN THE INFORMATION SOCIETY ;
INTERNATIONAL JOURNAL, SPRINGER,
BERLIN, DE, vol. 8, no. 4, 5 March 2009
(2009-03-05), pages 241-257, XP019744032, ISSN:
1615-5297, DOI: 10.1007/S10209-009-0149-0**

• **HANSEN D W ET AL: "In the Eye of the Beholder:
A Survey of Models for Eyes and Gaze", IEEE
TRANSACTIONS ON PATTERN ANALYSIS AND
MACHINE INTELLIGENCE, IEEE COMPUTER
SOCIETY, USA, vol. 32, no. 3, 1 March 2010
(2010-03-01), pages 478-500, XP011280658, ISSN:
0162-8828, DOI: 10.1109/TPAMI.2009.30**

• **VILLANUEVA A ET AL: "A Novel Gaze Estimation
System With One Calibration Point", IEEE
TRANSACTIONS ON SYSTEMS, MAN AND
CYBERNETICS. PART B:CYBERNETICS, IEEE
SERVICE CENTER, PISCATAWAY, NJ, US, vol.
38, no. 4, 1 August 2008 (2008-08-01) , pages
1123-1138, XP011247739, ISSN: 1083-4419**

• **CHUANG ZHANG ET AL: "Gaze estimation in a
gaze tracking system", SCIENCE CHINA
INFORMATION SCIENCES, SP SCIENCE CHINA
PRESS, HEIDELBERG, vol. 54, no. 11, 29 April
2011 (2011-04-29), pages 2295-2306,
XP019966769, ISSN: 1869-1919, DOI:
10.1007/S11432-011-4243-6**

• **DAN WITZNER HANSEN ET AL: "Homography
normalization for robust gaze estimation in
uncalibrated setups", PROCEEDINGS OF THE
2010 SYMPOSIUM ON EYE-TRACKING
RESEARCH & APPLICATIONS, ETRA '10, 22
March 2010 (2010-03-22), page 13, XP055114249,
New York, New York, USA DOI:
10.1145/1743666.1743670 ISBN:
978-1-60-558994-7**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

Field of the invention

The present invention concerns a method and apparatus for estimating the gaze, i.e. the 3D direction at which a user is currently looking at.

Description of related art

As a display of attention and interest, gaze is a fundamental cue in understanding people activities, behaviors, and state of mind, and plays an important role in many applications and research fields. In psychology and sociology, gaze information helps to infer inner states of people or their intention, and to better understand the interaction between individuals. In particular, gaze plays a major role in the communication process, like for showing attention to the speaker or indicating who is addressed, which makes gaze highly relevant for Human Robotics Interaction (HRI). In another direction, in Human Computer Interfaces (HCI) gaze information coordinated with other user inputs can lead to the development of intuitive systems beneficial for instance for people with limited body mobility, or more generally for entering commands into computer systems.

For these reasons, computer vision based gaze estimation has been well studied.

Some accurate techniques rely on eye geometry and on pupil center-corneal reflections under infrared illumination for example. Those methods require specialized and usually costly infrared hardware.

Methods based on natural light methods have also been described. Some methods include a step of extracting geometric features of the eyes, for example an ellipse fitted to the pupil or more complex shapes incorporating the eyelids. Those methods rely on small features of the image, such as the position of the iris center or the exact shape of the pupil. Therefore, they require high contrast and high resolution images in order to distinguish and reliably extract those features.

A. Villanueva and al. disclose in "A geometric approach to remote eye tracking (Universal Access in the Information Society, Springer, 2009) a gaze estimation method that is based on a geometrical model. The method involves a step of applying refraction and projection algorithms to the pupil image. D. Witzner et al. resume in "In the Eye of the Beholder: A survey of Models for Eyes and Gaze "(IEEE Transactions on pattern analysis and machine intelligence, Vol.32, No.3, 2010) a list of eye models suitable for eye detection that could be used for gaze estimation.

A. Villanueva and al. disclose in "A Novel Gaze Estimation System With One Calibration Point "published in IEEE Transactions on systems, man and cybernetics-Part B (Vol.38, No.4, 2008) the design of a gaze tracking systems based on a geometric model. The geometric model is based on glints positions and pupil ellipse in the captured image.

To avoid this difficulty of tracking small image features, appearance based methods are also known. Those methods learn a direct mapping between an eye image to the gaze parameters and thus avoid local features tracking. For example, neural networks have been trained to detect gaze from an image. Those systems usually require thousands of training samples or in-session training to perform reliably. A drawback of existing appearance-based methods is therefore that they require large sets of training data to handle eye image variations due to person's specific appearance, head pose, scale, illumination and eyelids movements when learning a general mapping, or require (less) per session training data resulting in overfitting to the person and conditions used during the training phase.

It is therefore an aim of the present invention to solve or mitigate the drawbacks of prior-art solutions.

In particular, an eye tracking method which does not rely on special hardware or high resolution camera is needed. For example, in order to minimize intrusion and accommodate user's movement, remote cameras with wide field of view would be preferred but lead to the challenge of low resolution imaging.

More generally, an eye tracking method based on consumer hardware would be advantageous.

It is another aim of the present invention to provide a method and an apparatus which do not rely on the identification and tracking of image features, thus being able to address low-resolution sensing while maintaining high-accuracy for mid to high resolution.

It is another aim of the present invention to provide a method and an apparatus which do not require large amounts of training data.

It is another aim of the present invention to provide a more accurate gaze estimation method and apparatus.

It is another aim of the present invention to provide a method and an apparatus which is based on a geometric model of the eye, and thus can be more easily adapted to new users or new ambient conditions, by extrapolating from existing data.

It is another aim of the present invention to provide a method and an apparatus which can be adapted to the given user and conditions from minimal or no effort from the user.

Brief summary of the invention

According to the invention, these aims are achieved by means of a method for estimating a gaze direction at which a user is looking at, comprising before a test phase (b):

a1) a step of retrieving a geometric model of at least one user's eye, defined by a set of parameters (U);
a2) retrieving an image observation model (f) expression how likely is a given image or a set of images of the eye given said geometric model for each configuration of values of the set of parameters (U);

and then, during the test phase:

b1) obtaining observation data (X, A) from at least one image, each image including at least one user's eye;
b3-b4) using the image observation model, whose parameters (U) include the visual and/or optical axis of the eye (o, v), determining the visual and/or optical axis of the eye (o, v) which is most likely to produce said observation data;
(b5) using said visual and/or optical axis to retrieve said gaze direction.

This geometric model could comprise further statistics or information, such as the standard deviation of the eyeball size for a given population.

According to another aspect of the method, the geometric model of the eye may be described with user specific parameters.

The user specific parameters may be determined for example during a calibration phase.

The user specific parameters may be derived from person independent geometric parameters and/or the calibration data.

The method may further comprise a step of retrieving, before said test phase, an image observation model expressing how likely is a given image or set of images of the eye given said geometric model.

The image observation model may be predefined.

The image observation model may be defined during a supervised, semi-supervised or unsupervised, calibration phase based on a plurality of training images of eyes.

The image observation model may be adapted during the test phase.

The appearance parameters in the image observation model may depend on the geometric model.

The method does not rely on identification of specific images features (such as iris center, or iris ellipse fitting), but on a global fit of a given eye geometric model to the observation data (corresponding to the captured image or/and filtered versions of it) through the image observation model. A subset of the geometric model could have been made user-specific during the calibration phase (such as the user-specific eyeball size). Therefore, this method avoids the need of local features estimation which has been, so far, a requirement for geometric based methods.

Similarly to appearance-based methods, the method thus uses the full captured eye image to infer the gaze parameters. However, rather than using a direct mapping from the eye image to gaze parameters, it interpret the visual data using an image observation model allowing to evaluate how well does a given geometric configuration (e.g. eyeball size, eye orientation, etc.) fit the visual data. In other words, the method thus uses an appearance geometric generative process to model eye images.

The method thus finds an adequate geometric configuration, including the gaze direction (defined by the visual and/or optical axis orientation), by optimizing the image observation model on the data (image and/or filtered versions of it).

This formulation circumvents the detection of local features, which are required in prior geometric based methods.

The method presents important advantages :

(i) it can be adapted to a given user from fewer training samples. The training samples are only needed to establish a user-specific model of the eye. A user-independent geometric model could have been established from training samples from different individuals and/or from statistical reports for a given population. This user independent geometric model could the n be adapted to a specific user and made user specific.
(ii) In one embodiment, the method could be adapted to different illumination conditions as the actual color observations become independent from the geometry. In order to achieve this, a parametric segmentation of the image of the eye could be an element of the image observation model.
(iii) the method can better handle low-resolution imaging as compared to feature tracking method (eg. ellipse fitting) while providing good results with mid-resolution
(iv) the method, as it is based on the geometric description of the eyeball, can handle gaze directions not seen in the calibration phase. Thus it can extrapolate from the calibration data allowing for less restrictive calibration procedures.

Therefore, the method is well adapted to gaze detection and gaze tracking from images captured with consumer cameras, including for example consumer RGB-D cameras.

The eye geometrical model may comprise a plurality of parameters, for example all the parameters, among the following lists:

eyeball rotation center ($p_c$);
visual axis offset (k);
nodal point distance from $p_c$ (d);
eyeball radius ($r_e$);
cornea radius ($r_c$);
left and/or right eye corners ($k_l$, $k_r$).

The set of parameters defining tge geometric model of the user's eye may be determined during a supervised, semi-supervised or unsupervised, calibration phase based on a plurality of training images of eyes.

The training images of the user's eye may comprise or be otherwise associated with additional information, such as the exact point of regard, or additional data which can be used to derive how likely it for the user to be gazing at a given point. For example, the training images of the user's eye may be associated with another images, audio data, interaction with a computer, external data, etc, which could be used to define a probability of looking at certain points. For example, a person is likely to look at another at key moments while the second person talks.

In one example, the user specific parameters are based on a plurality of 3D images, such as for example RGB-D images, captured with a RGB-D sensor such as, for example, a Kinect, Asus Xtion Pro Live or Carmine 1.09 from PrimeSense sensor.

Other types of images including depth information could be used, including for example stereoscopic, plenoptic or true 3D images.

The determination of the user's gaze direction in the test image captured during the online phase thus involves a fitting of the observation data (including for example a test image, and/or data extracted, associated or filtered from the image, such as edges) with a specific instance of the geometric model, corresponding to a particular orientation of the optical and/or visual axis of the eye. The fitting is conducted by evaluating on the data, using the image observation model, how adequate is a given geometric configuration which includes the eyeball orientation.

In a preferred embodiment, the geometric configuration which better fits the observation data is retrieved using an optimization procedure of the image observation model evaluated on the data.

In one embodiment, the eyelid opening is also used as parameters in the model.

This method based on a generative model is well adapted to the detection of eye gaze in low quality images, and/or in varying conditions of capture.

This method is also easier to adapt to different users, and require less training than prior art methods based on machine learning.

In a preferred embodiment, the fit of the geometric model to the observation data uses a parametric segmentation of the user's eye in a plurality of semantic regions. The parametric segmentation establishes a link between the eyeball geometry and the observed image domain coordinates.

A semantical segmentation means in this context a segmentation of the image into regions depending on their function or appearance differentiation. For example, the semantic regions may include a plurality of regions among a group including cornea, sclera (white of the eye) and skin (including eyelid).

The use of semantic regions allows decoupling the gazing process and user geometry from the ambient conditions (color appearance - white balance).

The use of semantic regions, as a by-product of the image observation model, further avoids the difficult task of identifying or tracking specific features, for example the iris center.

The segmentation depends on observed color or gray scale level in each of said semantic regions.

The color of each pixel of the semantic regions in the eye's model may depend on this segmentation. For example, the color of each region may depend on session, or even image dependent color models, i.e. colors models determined from the image captured during the test phase. This allows a fitting of the user-specific geometric model with the captured image which is more independent from the lighting conditions during the online capture, and thus more reliable. Therefore, the ambient conditions are decoupled from the user specific geometry.

In one preferred embodiment, the test phase includes a rectification of the captured image, in order to produce a rectified image as if it would have been seen from a canonical head pose, such as a frontal head pose. This solves the head pose-dependent eye appearance problem, and makes it possible to infer gaze independently of the head pose and facilitates the definition of the parametric eye image segmentation function.

The rectification may include a step of fitting a user specific 3D facial model with the image captured during the test phase, and warping the texture according to this model and the head pose estimated by the fitting.

Alternatively, the rectification step may include warping the image according to depth measurements and an estimate of the head pose.

The image observation model may be defined as a generative probabilistic model in which the geometric configuration, including the eyeball orientation, are probabilistic latent variables. The estimation of how likely it is for a given image or set of images to have been generated by a given geometric configuration is defined by the posterior distribution of the geometric parameters given the observed data.

This generative probabilistic model may also comprise appearance parameters as latent variables. These may include parameters of the color distributions associated to the semantic regions within the segmentation, thus the inference also determines the per-image color model parameters associated to the semantic segmentation regions.

The fitting of the geometric model to the observation data may be done through an optimization of the image observation model, to find an adequate geometric configuration. This may comprise an approximate inference methodology to find the posterior distribution on the latent variables or specific values, such as the maximum-a-posteriori or maximum-likelihood geometric configuration and/or appearance parameters.

The approximate inference may comprise a variational inference technique such that an approximation to the posterior distribution is found.

The method for image model optimization may provide a notion of uncertainty on the estimated geometric parameters.

The fitting of the observation data with a particular instance of the geometric model gazing at a particular direction may involve a steps of fitting the eyelids opening and the orientation of the eye's optic axis. The method may comprise a step of determining the visual and/or optical axis of the eye which is most likely to produce said observation data uses images including the two eyes, and a constraint on the relation between the movements of the two eyes.

The method may comprise a step of determining the visual and/or optical axis of the eye in one image by using a knowledge of this visual and/or optical axis in previous or future images of a same sequence.

The invention is also related to an apparatus for estimating a user's gaze direction, comprising the following components:

a camera for capturing a user's face;
a database storing user-specific eye and facial parameters;
a computing system comprising a memory having stored thereon a computer program comprising an algorithm capable of perform the method described in the present application.

The invention is also related to a computer readable storage medium having recorded thereon a computer program, the computer program comprising an algorithm capable of perform the method described in the present application.

Brief Description of the Drawings

The invention will be better understood with the aid of the description of an embodiment given by way of example and illustrated by the figures, in which:

Fig. 1 is a flow chart showing the main steps of a method according to the invention.

Fig. 2 is a flow chart showing the main steps of a test phase according to the present invention.

Fig. 3 is a flow chart detailing the rectification step according to the present invention.

Fig. 4 depicts schematically illustrates examples of images which can be used at various steps of the invention. Part a) of the figure shows images used during the calibration phase, i.e., offline, while part b) shows example of images used during the test phase (online).

Fig. 5 illustrates a step of generating user's specific, segmented images of one user's eye.

Fig. 6 schematically illustrates the geometry of one eye.

Fig. 7 illustrates the gaze direction vector o in a three-axis coordinate system.

Detailed Description of possible embodiments of the Invention

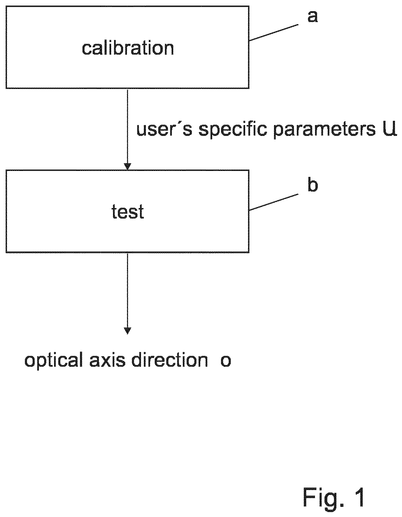

Figure 1 is a flowchart that schematically illustrates two main steps a and b of the method according to an embodiment of the invention. Step a is a phase during which a geometric model of at least one user's eye is retrieved; this geometric model could be defined by a set of parameters U. In addition, the parameters (such as A) of an image observation model f expressing how likely is a given image or set of images of the eye given said geometric model may be retrieved.

Those initial steps in phase a may be performed offline, preferably for each user. In one embodiment, the geometric model and the image observation model may be retrieved from a set of images of the user eye gazing at a known direction, for example during an explicit calibration session. The calibration may be supervised, unsupervised, or partially supervised.

Alternatively, those initial steps a may be performed implicitly without the user knowing or doing an explicit action. The user geometric model and/or the image observation model may also be retrieved from images of the user eye captured during the online session, i.e., without any explicit calibration session.

The geometric model of the user eye may also be retrieved without any image of the user eye; for example, a user independent model could initially be used, for example a model generated from a plurality of eye images from different users. In one embodiment, the geometric model is generated by interpolating models from different users sharing some common features with the user (for example sex, age, ethnicity; etc). In another embodiment, the geometric model is user independent and defined by a set of geometric statistics, which can be retrieved from prior studies or from the training data for a set of users.

Similarly the parameters of an image observation model may be retrieved from images of the user's eye, or without any image of the user eye; for example, the color distributions of different semantic regions of the eyes can be learned offline from a plurality of eye images of other persons.

This user's eye model is characterized and defined by user specific parameters $u = \{p_c, r_e, r_c, k, d, k_{lr}\}$ which define the fixed eye geometry. The notations are defined in Table 1:

Table 1

| Symbol | Description |
|---|---|
| $I; (u, v)$ | Image *index* and pixel coordinates |
| $p_c$ | Eyeball rotation center |
| $k = (\phi_k, \theta_k)$ | Visual axis deviation |
| $d$ | Nodal point distance from $p_c$ |
| $a := (k, d)$ | Axial parameters |
| $r_e, r_c$ | Eyeball and cornea radii |
| $k_{lr} = (k_l, k_r)$ | Left and right eye corners |
| $k_l = (k_{lu}, k_{lv})$ | Left eye corner in image coordinates |
| $k_r = (k_{ru}, k_{rv})$ | Right eye corner in image coordinates |
| $s := (r_e, r_c, k_l, k_r)$ | Structure parameters |
| $p$ | Visual target point location |
| $o = (\phi, \theta)$ | Optical axis orientation |
| $u_e, l_e$ | Upper and lower eyelid opening |
| $m := (o, u_e, l_e)$ | Movement parameters |
| $\wedge_l$ | Class *l* color distribution parameters |
| $c$ | Observed color at pixel *u, v* |
| $\lambda \in \{0,1\}$ | Occlusion state for pixel *u, v* |

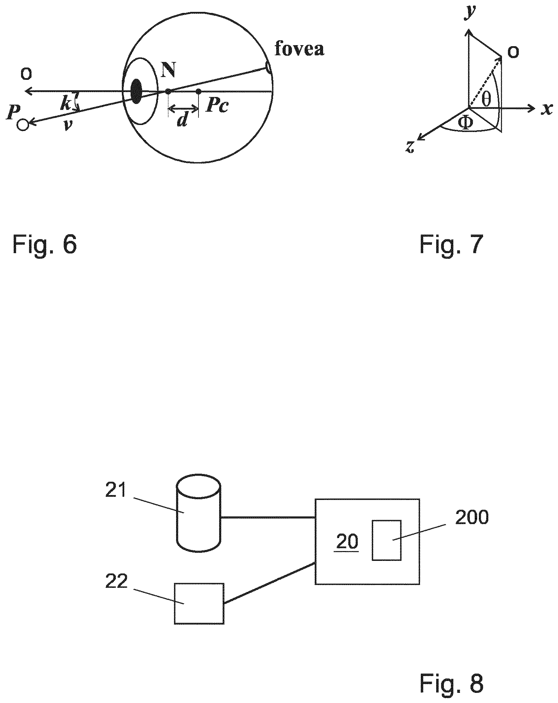

Figure 6 illustrates the eye geometry and the definition of the optical and visual axis **o,** v when the eye is looking at a target p. The process of gazing a visual target p consists of rotating the eyeball around the point $p_c$ such that the visual axis v intersects p. The visual axis v is the line connecting the fovea (i.e. the point of highest visual acuity in the retina) and the nodal optical point N. It differs from the optical axis o which is the line connecting the center of the rotation $p_c$ and the pupil center. The two axis o, v are parameterized by the angles $\phi$ and $\theta$ in the coordinate system x,y,z as shown on Figure 7. As the eye is a rigid body, the difference between these axis is fixed and can be represented by the person dependent angles $\kappa = (\phi_\kappa, \theta_\kappa)$ such as v = o + k

Thus, if the axial parameters a = (k, d) are known, then the eye rotation o can be defined as a user's specific function of the position of the target p.

In this embodiment, an image observation model is defined from a probabilistic generative process which depends on the geometric model. The generative process is as follows:

i) a user-specific eye geometry (defined by parameters U) is first drawn from a user independent geometric model defined as a probability distribution over the likely human eyeball possible geometries;

ii) then, per sample image, an eye orientation $(o, v)$ and eyelid opening $(u_e, l_e)$ is drawn from a prior distribution. The eyeball orientation will be constrained by the position p of the visual target, if it is available. The eyelids opening may be constrained by the drawn eyeball orientation as well;

iii) whether a pixel is an outlier (due to, for example, occlusions or missing data) is drawn from a Bernoulli distribution;

iv) given the drawn geometry (including user-specific geometric parameters, gaze and eyelids opening) and outliers flag, a semantic segmentation of the eye image is computed from the parametric segmentation function;

v) given the segmentation, for each pixel, a color value is drawn from the color distribution defined for the semantic region the pixel belongs to.

Note that the color distribution are parameterized by the parameters A. In the framework, these parameters could be drawn as well (in the probabilistic sense) from prior distributions.

The above image observation models allows to define an image likelihood (from the individual pixel color likelihoods) and thus to define how well a geometric eye configuration fits the data, or more generally, how well, some parameters (of the geometric model, of the appearance model) fit the data, assuming the other parameters are given.

The final goodness of fit of the image observation model can be defined by combining the image likelihood with prior knowledge over parameters, e.g. through distributions over the geometrical parameters. That is, the goodness of fit of the image observation model is defined by the posterior distribution of the (geometry) parameters given the observation data. The image observation model may evaluate the geometry for more than one image jointly, such that information is shared between the images to find more adequate solutions.

Therefore, the user's specific model of the user's eye could be determined from a sufficient number of training images of the eye acquired during the calibration phase. Since the posterior distribution is difficult to derive analytically, a Variational Bayes formulation can be used as an approximate inference technique to finds the optimal geometric configuration (parameters V) which best explains the data in terms of observations and prior distributions as explained above.

The inference process can benefit as well from knowing the corresponding target location p for a set of training images. The known target location is thus added to part of the observed data.

The initial step a, in this embodiment, preferably also includes a step of generating from a few images, for example 3D images, a 3D mesh model of the user's head. This facial model will be used for head pose identification and tracking, as will be described later.

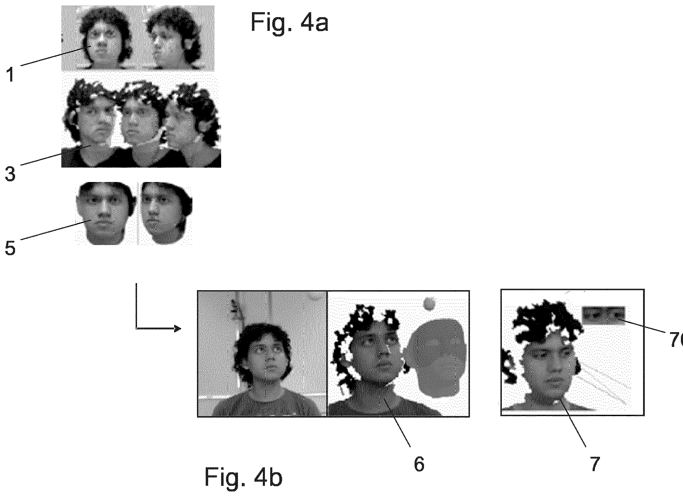

This generation of a user specific 3D facial model 5 is schematically illustrated on Figure 4a illustrates. The 3D is generated from a plurality of images, such as RGB-D images 1, of the user's face seen from various positions. This process may involve a fitting of a 3D Morphable Model (3DMM) to RGB-D data which may also rely on corresponding points in the multiple instances (3). This results in a user specific facial shape mesh (image 3).

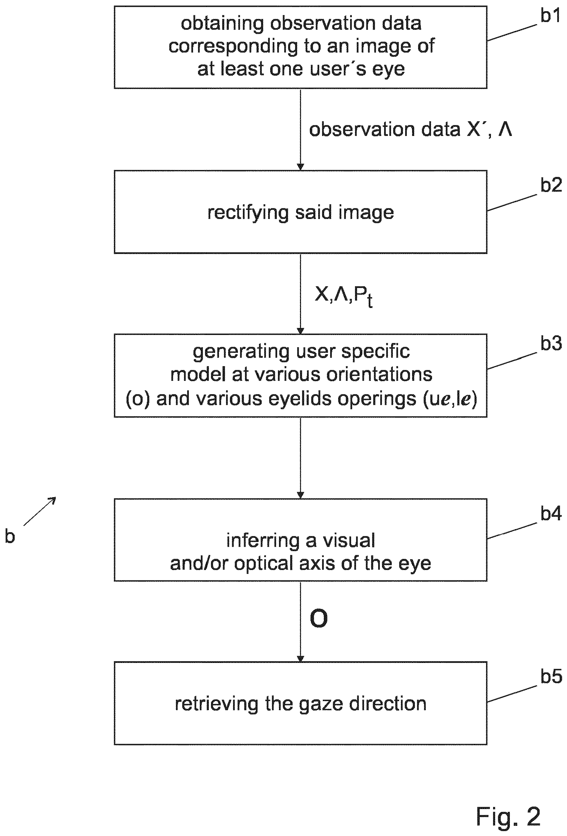

Figure 2 is a flowchart illustrating some steps of a test phase b according to an embodiment of the present invention. This test phase is performed online; its purpose is to infer the direction of the optical axis o (or eyeball orientation), and eventually the 3D direction at which the user is looking, based on the previous calibration, head pose, and on captured images of the user.

More precisely, this test phase b allows to retrieve image specific parameters $V = \{o, u_e, l_e\}$ related to the actual gaze activity : what is the person's eye orientation (characterized by $o$ and/or $v$) and how are the eyelids open ($u_e, l_e$). The parameters A of a color distribution model could be defined for the given session, obtained from the calibration phase or retrieved (estimated) from the specific image, as it depends on the ambient conditions such as the white balance and brightness for example

During step b1, at least one image of the user face is captured with a camera, such as without limitation a RGB-D camera such as a Microsoft Kinect, Asus Xtion Pro Live or Carmine 1.09 from PrimeSense camera. The image includes at least one user's eye, or preferably both users' eye. The image is represented by observation data X', for example by an image file or dataset in a RGB-D format. The image could be a still image or a frame as part of a video sequence. A indicates the color distribution parameters of this image.

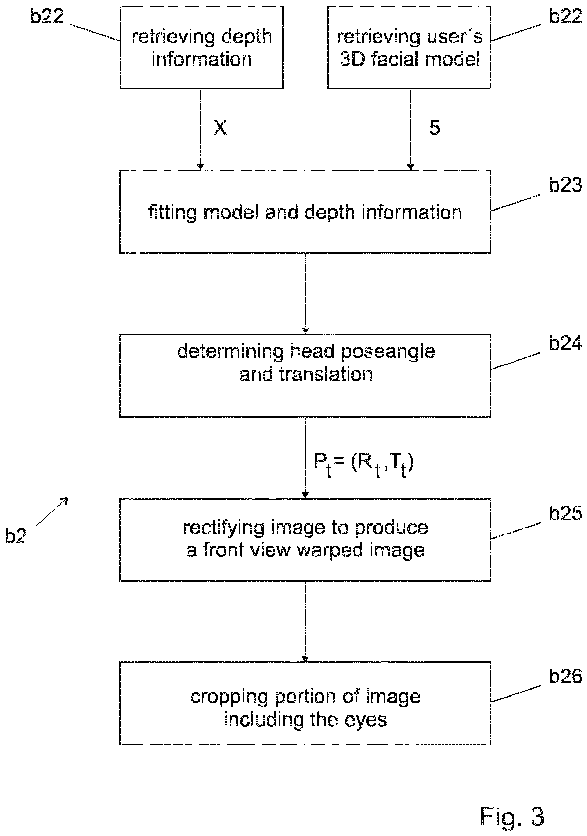

At step b2, this image is rectified to retrieve a head-pose independent image, represented by the (rectified) observation data X'. This step is optional. It will be described in more details in relation with Figure 3. Briefly, the aim of this step is to produce from the image data X a rectified facial image as if the head was static and in front of the camera, i.e., as seen from a frontal head pose. This is done by fitting, frame-by-frame, the personalized 3D mesh template obtained during the calibration phase to depth data in X', using iterative closest points. This results for each frame t in a 3D rotation and translation vector $\mathbf{p}_t=\{\mathbf{R}_t, \mathbf{t}_t\}$. The rectified facial image is obtained by transforming the RGB-D data X to a textured 3D mesh , and then re-rendering the texture using the inverse head pose parameters $\mathbf{p}_t^{-1} = \{\mathbf{R}_t^T, -\mathbf{R}_t^T\mathbf{t}_t\}$.

At step b3 and b4, image specific parameters $V = \{o, u_e, l_e\}$, including the optical axis orientation o referred to the head coordinate system can then be determined from this rectified 3D Mesh Model represented by X'.

More precisely, at step b3, the variational Bayes technique is used to optimize the image observation model and finding the geometric parameters which explain better the data X'. The found parameters are $V = \{o, u_e, l_e\}$.

The user specific model decouples the geometry to the ambient conditions, thanks to the semantic segmentation and the color distributions. The segmentation separates an eye image into semantic regions (or classes), preferably into three regions: the cornea (composed of the pupil and iris), the sclera (or white of the eye) and the skin. A different number of regions, for example by distinguishing between pupil and iris, and between the eyelid and the skin around the eye, could also be considered for example if high resolution images are available.

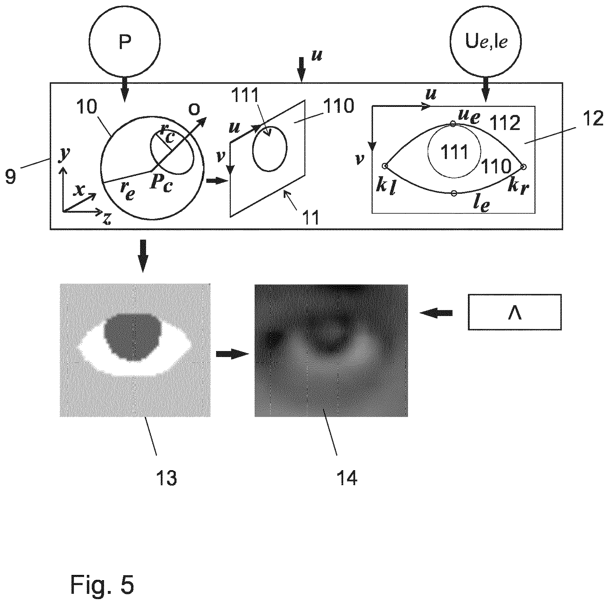

The rectangle 9 in Figure 5 illustrates the parametric segmentation process: assuming that a user specific eye model 10 defined by the user eye geometric parameters U is known, then each eye orientation o defines a cornea 111 -sclera 110 segmentation 12, obtained as an orthogonal projection 11 of the 3D cornea contour into the xy plane, followed by a transformation to image coordinates uv. The reference 112 designates the skin region, including the eyelids.

To define the segmentation of the skin region, it is possible to rely on a subset of the parameters U characterizing the eyelids structure (eye corners $k_l$ and $k_r$) and another set controlling the eyelids opening. In one embodiment, the upper and lower eyelids are modeled with quadratic Bezier curves sharing the eyelids corners. The vertical position of the control points $u_e$ and $l_e$ define the opening of the eyelids, and thus, the skin segmentation.

It is, therefore, possible to generate user specific segmented images, such as images 13 on Figure 5, of a user eye gazing at any particular direction defined with parameter v and with various eyelid openings defined by $u_e$, $l_e$.

The observation data corresponding to the captured image may include a fourth class λ of region, corresponding to outliers, such as for example missing data, occlusions and specular reflections.

The color distribution λ associated to one class can be defined as $p(c|\wedge_l)$. The likelihood of a color pixel in the observation data X' is then simply defined as the likelihood given its class (either an outlier, or one of the three eye region classes).

This segmentation is used to generate color eye images 14 on Figure 5 by combining the segmented model 13 with image dependent color model distributions A for defining the color of each region. For example, color samples may be acquired from one or several images in order to estimate the color model parameters that will be used for those images, or for a complete session. This allows for adaptation to different illumination and contrast conditions.

Table 2 illustrates an example of algorithm for the geometric generative gaze estimation. It could be described as a stochastic extension of the process of gazing up to the generation of user-specific and ambient conditions specific eye images, under which some geometric parameters are defined as a random variable. We can denote by

$$x \sim \mathcal{N}(\mu_x, \sigma_x)$$

a random variable x being drawn from a Gaussian distribution with mean $\mu_x$ and standard deviation $\sigma_x$, , and the "hat" (^) notation to represent the hyperparameters of a prior distribution.

Table 2

| |
|---|
| • Draw the eyeball rotation center $p_c$: $$p_c \sim \left(\mathcal{N}(\hat{\mu}_{p_{cx}}, \hat{\sigma}_{p_{cx}}), \mathcal{N}(\hat{\mu}_{p_{cy}}, \hat{\sigma}_{p_{cy}}), \mathcal{N}(\hat{\mu}_{p_{cz}}, \hat{\sigma}_{p_{cz}})\right)$$ • Draw axial parameters $a := (\kappa, d)$: $$\kappa \sim \left(\mathcal{N}(\hat{\mu}_{\phi_\kappa}, \hat{\sigma}_{\phi_\kappa}), \mathcal{N}(\hat{\mu}_{\theta_\kappa}, \hat{\sigma}_{\theta_\kappa})\right)$$ $$d \sim \mathcal{N}(\hat{\mu}_d, \hat{\sigma}_d)$$ • Draw structure parameters $s := (r_e, r_c, k_l, k_r)$: $$r_e \sim \mathcal{N}(\hat{\mu}_{r_e}, \hat{\sigma}_{r_e})$$ $$r_c \sim \mathcal{N}(\hat{\mu}_{r_c}, \hat{\sigma}_{r_c})$$ $$k_l \sim \left(\mathcal{N}(\hat{\mu}_{k_{lu}}, \hat{\sigma}_{k_{lu}}), \mathcal{N}(\hat{\mu}_{k_{lv}}, \hat{\sigma}_{k_{lv}})\right) \quad k_r \sim \left(\mathcal{N}(\hat{\mu}_{k_{ru}}, \hat{\sigma}_{k_{ru}}), \mathcal{N}(\hat{\mu}_{k_{rv}}, \hat{\sigma}_{k_{rv}})\right)$$ |

(continued)

• For each image $I$ = 1,...,$N$:
- Draw the visual target $p \sim$ *uniform*
- Draw movement parameters m := (o, $u_e$, $l_e$):

$$\star \quad o \sim (\mathcal{N}(f_\phi(p; a, p_c), \hat{\sigma}_o), \mathcal{N}(f_\theta(p; a, p_c), \hat{\sigma}_o))$$

$$\star \quad u_e \sim \mathcal{N}(a_u \theta + b_u, \hat{\sigma}_{u_e})$$

$$\star \quad l_e \sim \mathcal{N}(\hat{\mu}_{l_e}, \hat{\sigma}_{l_e})$$

- For each ($u, v$) = [1,..., *width*], [1,...,*height*]:
  * Draw outlier or not indicator $\lambda \sim$ *Bernoulli*($\hat{\lambda}$)
  * Draw pixel color c $\sim p_{u,v}(c|\lambda, m, s, p_c, \{\wedge_I, ]_I)$

In one embodiment, the upper eyelid is correlated with the elevation angle of the eye by means of a linear Gaussian model. This encodes the effect of the upper eyelid following the vertical orientation of the eye.

Prior distributions have a semantic and/or anatomical interpretation. Therefore the hyperparameters are fixed to values that can be found in the literature (e.g. $r_e \approx$ 12mm) or are a consequence of the pose-rectification processing (e.g. it is known where the eye corners are expected to be from the eye image cropping).

At step b4 of Figure 2, the visual axis v and/or the optical axis o are inferred for a given captured image, described by the rectified parameters X. As already noted, the optical axis o and the visual axis v are related by a fixed, user-dependent relation.

Let Z be the latent variables to be inferred in the method of table 2. As already mentioned, X describes the observed data. It might not be possible or fast to estimate analytically the posterior distribution p(Z|X). Therefore, in one embodiment, a Variational Bayes method is used to approximate Z, using a distribution $q(Z)$.

This leads to the definition of the variational lower bound $\mathcal{L}(q)$, a functional whose maximization with respect to $q$ is equivalent to a minimization of the Kullback-Leibler divergence between q(Z) and p(Z|X). The optimal q*(Z) is then used as a substitute of the posterior. The following distribution q(Z) could be used, using the parametric form:

$$q(Z) = \mathcal{N}(\mu_d, \sigma_d) \mathcal{N}(\mu_{\phi_\kappa}, \sigma_{\phi_\kappa}) \mathcal{N}(\mu_{\theta_\kappa}, \sigma_{\theta_\kappa}) \mathcal{N}(\mu_{r_e}, \sigma_{r_e})$$
$$\mathcal{N}(\mu_{r_c}, \sigma_{r_c}) \mathcal{N}(\mu_{p_{cx}}, \sigma_{p_{cx}}) \mathcal{N}(\mu_{p_{cy}}, \sigma_{p_{cy}}) \mathcal{N}(\mu_{p_{cz}}, \sigma_{p_{cz}})$$
$$\mathcal{N}(\mu_{k_{lu}}, \sigma_{k_{lu}}) \mathcal{N}(\mu_{k_{lv}}, \sigma_{k_{lv}}) \mathcal{N}(\mu_{k_{ru}}, \sigma_{k_{ru}}) \mathcal{N}(\mu_{k_{rv}}, \sigma_{k_{rv}})$$
$$\prod_I \left[ \mathcal{N}(\mu_\phi, \sigma_\phi) \mathcal{N}(\mu_\theta, \sigma_\theta) \mathcal{N}(\mu_{u_e}, \sigma_{u_e}) \mathcal{N}(\mu_{l_e}, \sigma_{l_e}) \prod_{u,v} q(\lambda) \right]$$

.

Every continuous variable could be been defined as a univariate Gaussian. The motivation for this q(Z) is that it is possible to compute the derivatives of $\mathcal{L}(q)$ with respect to the Gaussian parameters. We can then compute the derivatives using Monte Carlo expectations to address the complex relations in our model. A factorized $q$(Z) also allows to optimize $\mathcal{L}(q)$ in an iterative fashion, where one factor is optimized at the time, leading to an increase of $\mathcal{L}(q)$ until global convergence.

During this test phase, there is no knowledge on the position of the visual target location p, but the parameters V defining the geometry of the eye are known. In this case the purpose of the inference algorithm is to infer the parameters o (optical axis) and $u_e$, $l_e$ (upper and lower eyelid opening), given one or a plurality of captured images.

Finally, at step b5 of Figure 2, the gaze direction in a world coordinate system is retrieved by transforming optical axis o in the head coordinate system, using the rotation and translation parameters $p_t$={$R_t$, $t_t$}.

Figure 3 illustrates an embodiment of step b2 for rectifying the captured image and generate a head-pose independent image defined by a set of parameters X and a pose vector $p_t$. During the capture phase, the method involves to retrieve at step b21 a set of image specific depth information from the non rectified observation data X'. At step b22, a user's specific 3D facial model 5 (generated during the calibration phase) is retrieved, such as a 3D mesh model (3DMM). At step 23, the depth information in observation data X' is registered at each instant with this model, using known registration methods, as illustrated with image 6 in Figure 4b. It is therefore possible at step b24 to retrieve the head pose angle $\mathbf{R}_t$ and translation $\mathbf{t}_t$. Those parameters are used at step b25 to produce a rectified image 7, defined by rectified observation data X, corresponding to a front view warped image. The portion 70 of the rectified image 7 that includes the eyes can then be cropped at step b26.

The rectification method described in relation with Figure 3 thus relies on depth information in the observation data X' in order to fit a 3D mesh model. In another embodiment, a 3D head pose tracker based on image data only (not depth), e.g. using Active Appearance Models, could be used. Therefore, the method could be used when depth data is not available, i.e. for standard monocular cameras. In this case, a head pose dependent parametric segmentation function could be used to measure the image likelihood, but it would still infer the geometric eyeball parameters referred to the head fixed coordinate system.

In order to infer and/or track the gaze direction of unknown users (i.e. new users who haven't performed a calibration step), it is possible to replace the use of user images during the calibration phase and to use instead a set or pre-collected gaze appearance models from different users, which combined can defined a user independent geometric model, based on statistics among the set of users.

One might for example consider an interpolation of the the appearance of an user unknown to the system from existing models. In one embodiment, a subset of models adequate for the current user is used, ignoring the rest and thus resulting in faster computation.

The above method could be used to infer the gaze direction from images of a single eye, or more reliably by using images including the two eyes. In this later case, a constraint on eye movements (left and right eye gaze always at a single point) could be used in order to perform a faster and more precise gaze estimation.

Similarly, gaze dynamics temporal information could be exploited to improve the accuracy of the gaze estimates. The distribution of eye movements (fixations, saccades, etc.) is known from other works and could be used as a prior to improve the gaze accuracy under temporal observations. For example, the likelihood of a particular visual target location p for a given frame could depend on the location of p in previous and future frames.

Instead of using a deterministic position of the visual target p, another embodiment could rely on a calibration phase in which p is a random variable which models the probability of looking a point among a set of possible visual targets locations. The probability distribution could be defined from external information, such as visual attention maps, speech activity from the interaction among a group of users, from expected points of visual attention from a user towards a robot, etc.

Figure 7 schematically illustrates an apparatus for estimating a gaze direction at which a user is looking at. The apparatus may include a computing system 20, such as a personal computer, a server, a smartphone, a wearable computer, an optical head-mounted display (such as Google Glass for example), a tablet, a TV-set, or any type of microprocessor-based system. An image capture element 22, such as a camera, a webcam, a RGB-D sensor, a plenoptic camera, or any kind of image-capture based sensor, is connected or integrated to this computing system for producing images of a user including his face with eye portions. Furthermore, a database 21 is used as part of the computing system 21, or connected to this computing system, for storing user-specific parameters, such as a 3D mesh model of his phase and a geometric model of at least one of his eyes. This database may be stored locally in the computing system, or remotely for example on a remote server. In its simplest form, this database may comprise a single file with user specific parameters, such as a text or XML file, or be part of a more complex database system, such as a SQL database for example. The computing system 20 further includes a computer readable storage medium having recorded thereon a computer program, the computer program comprising an algorithm capable of perform the above described method.

It is to be recognized that depending on the embodiment, certain acts or events of any of the methods described herein can be performed in a different sequence, may be added, merged, or left out altogether (e.g., not all described acts or events are necessary for the practice of the method). Moreover, in certain embodiments, acts or events may be performed concurrently, e.g., through multi-threaded processing, interrupt processing, or multiple processors, rather than sequentially.

Various modifications and variations to the described embodiments of the invention will be apparent to those skilled in the art without departing from the scope of the invention as defined in the appended claims. Although the invention has been described in connection with specific preferred embodiments, it should be understood that the invention as claimed should not be unduly limited to such specific embodiment.

**Claims**

1.  A method for estimating a gaze direction at which a user is looking at, comprising before a test phase (b):

    a1) a step of retrieving a geometric model of at least one user's eye, defined by a set of parameters (U);
    a2) retrieving an image observation model (f) expressing how likely is a given image or a set of images of the eye given said geometric model for each configuration of values of the set of parameters (U);

    and then, during the test phase (b):

    b1) obtaining observation data (X, A) from at least one image, each image including at least one user's eye;
    b3-b4) using the image observation model, whose parameters (U) include the visual and/or optical axis of the eye (o, v), determining the visual and/or optical axis of the eye (o, v) which is most likely to produce said observation data;
    (b5) using said visual and/or optical axis to retrieve said gaze direction.

2.  The method of the claim 1, wherein said image observation model (f) is adapted during said test phase (b).

3.  The method of the claim 1 or2, wherein at least a subset of said parameters (U) is determined during a supervised, semi-supervised or unsupervised, calibration phase based on a plurality of training images of eyes.

4.  The method of claim 3, wherein said training images comprise or are associated with additional information used to derive how likely it for the user to be gazing at a given point.

5.  The method of one of the claims 1 to 4, using an implicit or explicit segmentation of the user's eye image into a plurality of semantic regions; preferably, said semantic regions including a plurality of regions among a group including cornea, sclera and skin; preferably , wherein the observed color or gray scale level in each of said semantic regions of the eye depends on said segmentation.

6.  The method of one of the claims 1 to 5, further comprising:
    b2) rectifying said image as if it would have been seen from a canonical head pose;
    preferably said step of rectifying including fitting a user specific 3D facial model with said image.

7.  The method of one of the claims 1 to 6, said image including at least one user's eyes being a RGB-D, a stereoscopic, a plenoptic or a 3D image.

8.  The method of one of the claims 1 to 7, said set of parameters (U) comprising a plurality of parameters among:

    eyeball rotation center ($p_c$);
    visual axis offset (k);
    nodal point distance from $p_c$ (d);
    eyeball radius ($r_e$);
    cornea radius ($r_c$);
    left and/or right eye corners ($k_l$, $k_r$).

9.  The method of one of the claims 1 to 8, wherein said step of determining the most likely visual and/or optical axis comprises a determination of the most likely upper and/or lower eyelid openings.

10.  The method of one of the claims 1 to 9, wherein said observation model is based on a probabilistic or statistical model.

11.  The method of one of the claims 1 to 10, wherein said set of parameters (*U*) defining a geometric model of at least one eye is made user specific based on a plurality of training images of said user's eye.

12.  The method of one of the claims 1 to 11, wherein said step of determining the visual and/or optical axis of the eye **(o, v)** which is most likely to produce said observation data uses images including the two eyes, and a constraint on the relation between the movements of the two eyes.

13.  The method of one of the claims 1 to 12, wherein said step of determining the visual and/or optical axis (o, v) of the eye in one image involves a knowledge of this visual and/or optical axis in previous or future images of a same sequence.

14. An apparatus for estimating a user's gaze direction, comprising the following components:

> a camera (22) for capturing a user's face;
> a database storing user-specific eye and facial parameters (21);
> a computing system (20) comprising a memory having stored thereon a computer program comprising an algorithm capable of perform the method of any one of the claims 1 to 13.

15. A computer readable storage medium having recorded thereon a computer program, the computer program comprising an algorithm capable of perform the method of any one of the claims 1 to 13.

**Patentansprüche**

1. Ein Verfahren zum Schätzen einer Blickrichtung, in die ein Benutzer blickt, umfassend vor einer Testphase (b):

> a1) einen Schritt, ein geometrisches Modell, das durch einen Satz von Parametern (U) definiert ist, von mindestens einem Auge des Benutzers abzurufen,
> a2) Abrufen eines Bildbeobachtungsmodells (f), das ausdrückt, wie wahrscheinlich ein gegebenes Bild oder ein Satz von Bildern des Auges ist angesichts des geometrischen Modells für jede Konfiguration von Werten des Satzes von Parametern (U);

und dann, während der Testphase (b):

> b1) Erhalten von Beobachtungsdaten (X, A) aus mindestens einem Bild, wobei jedes Bild mindestens ein Benutzerauge enthält;
> b3-b4) Verwenden des Bildbeobachtungsmodells, dessen Parameter (U) die visuelle und/oder optische Achse des Auges (o, v) enthalten, bestimmen der visuellen und/oder optischen Achse des Auges (o, v), die am wahrscheinlichsten die Beobachtungsdaten erzeugt;
> (b5) Verwenden der visuellen und/oder optischen Achse zum Abrufen besagter Blickrichtung.

2. Verfahren nach Anspruch 1, wobei das Bildbeobachtungsmodell (f) während der Testphase (b) angepasst wird,

3. Verfahren nach Anspruch 1 oder2, wobei mindestens eine Teilmenge der besagten Parameter (U) während einer überwachten, einer halbüberwachten oder einer unüberwachten Kalibrierungsphase basierend auf einer Vielzahl von Trainingsbildern von Augen bestimmt wird.

4. Verfahren nach Anspruch 3, wobei die Trainingsbilder zusätzliche Informationen, die verwendet werden, um abzuleiten, wie wahrscheinlich es ist, dass der Benutzer auf einen gegebenen Punkt blickt, beinhalten oder mit besagten zusätzlichen Informationen assoziiert sind.

5. Verfahren nach einem der Ansprüche 1 bis 4, unter Verwendung einer impliziten oder expliziten Segmentierung des Augenbildes des Benutzers in eine Vielzahl von semantischen Regionen, wobei die semantischen Regionen vorzugsweise eine Vielzahl von Regionen aus einer Gruppe einschließlich Hornhaut, Sklera und Haut umfassen; wobei vorzugsweise die beobachtete Farb- oder Graustufenstufe in jedem der besagten semantischen Bereiche des Auges von der Segmentierung abhängt.

6. Verfahren nach einem der Ansprüche 1 bis 5, des Weiteren umfassend:
b2) Entzerren des Bildes, als ob es von einer kanonischen Kopfhaltung betrachtet würde, wobei der Schritt des Entzerrens vorzugsweise das Anpassen eines benutzerspezifischen 3D-Gesichtsmodells mit dem Bild beinhaltet.

7. Verfahren nach einem der Ansprüche 1 bis 6, wobei das Bild mindestens ein Auge des Benutzers, das ein RGB-D, stereoskopisches, plenoptisches oder 3D-Bild ist, beinhaltet.

8. Verfahren nach einem der Ansprüche 1 bis 7, wobei der Satz von Parametern (U) eine Vielzahl von Parametern umfasst, darunter:

den Augapfel-Rotationszentrum ($p_c$);
den Versatz der Sehachse (k);
den Nodalpunktabstand von ($p_c$) (d);
den Augapfel-Radius ($r_e$);
den Hornhautradius ($r_c$);
den linken und / oder den rechten Augenwinkel ($k_l$, $k_r$).

9. Verfahren nach einem der Ansprüche 1 bis 8, wobei der Schritt zur Bestimmung der wahrscheinlichsten visuellen und/oder optischen Achse eine Bestimmung der wahrscheinlichsten oberen und/oder unteren Augenlidöffnungen umfasst.

10. Verfahren nach einem der Ansprüche 1 bis 9, wobei besagtes Beobachtungsmodell auf einem probabilistischen oder einem statistischen Modell beruht.

11. Verfahren nach einem der Ansprüche 1 bis 10, wobei besagter Satz von Parametern (U, der ein geometrisches Modell von mindestens einem Auge definiert, gemäss einer Vielzahl von Trainingsbildern des Auges des Benutzers benutzerspezifisch gemacht wird.

12. Verfahren nach einem der Ansprüche 1 bis 11, wobei der Schritt zur Bestimmung der wahrscheinlichsten visuellen und/oder optischen Achse des Auges (o, v), das am ehesten die besagten Beobachtungsdaten erzeugt, Bilder verwendet, die die beiden Augen sowie eine Einschränkung in der Beziehung zwischen den Bewegungen der beiden Augen beinhalten.

13. Verfahren nach einem der Ansprüche 1 bis 12, wobei der Schritt zur Bestimmung der visuellen und/oder optischen Achse (o, v) des Auges in einem Bild eine Kenntnis dieser visuellen und/oder optischen Achse in vorherigen oder in zukünftigen Bildern einer gleichen Sequenz einbezieht.

14. Eine Vorrichtung zur Schätzung der Blickrichtung eines Benutzers, die folgende Komponenten umfassend:

   - eine Kamera (22) zum Erfassen des Gesichts eines Benutzers;
   - eine Datenbank, die benutzerspezifische Augen- und Gesichtsparameter speichert (21);
   - ein Computersystem (20) mit einem Speicher, in dem ein Computerprogramm, das einen Algorithmus umfasst, der in der Lage ist, das Verfahren nach einem der Ansprüche 1 bis 13 durchzuführen.

15. Ein computerlesbares Speichermedium mit einem darauf aufgezeichneten Computerprogramm, wobei das Computerprogramm einen Algorithmus umfasst, der in der Lage ist, das Verfahren nach einem der Ansprüche 1 bis 13 durchzuführen.

**Revendications**

1. Une méthode d'estimation de la direction du regard d'un utilisateur, comprenant avant une phase de test (b) :

   a1) une étape de récupération d'un modèle géométrique d'au moins un œil de l'utilisateur, défini par un ensemble de paramètres (U) ;
   a2) récupérer un modèle d'observation d'image (f) exprimant la probabilité qu'une image donnée ou un ensemble d'images de l'œil soit conforme audit modèle géométrique pour chaque configuration de valeurs de l'ensemble de paramètres (U) ;

et ensuite, pendant la phase de test (b) :

   b1) obtenir des données d'observation (X, $\Lambda$) à partir d'au moins une image, chaque image comprenant au moins un œil de l'utilisateur ;
   b3-b4) utiliser le modèle d'observation d'image, dont les paramètres (U) comprennent l'axe visuel et/ou optique de l'œil (o, v), qui détermine l'axe visuel et/ou optique de l'œil (o, v) qui est le plus susceptible de produire lesdites données d'observation ;
   (b5) utiliser ledit axe visuel et/ou optique pour récupérer ladite direction du regard.

**2.** La méthode selon la revendication 1, dans laquelle ledit modèle d'observation d'image (f) est adapté pendant ladite phase de test (b).

**3.** La méthode selon la revendication 1 ou 2, dans laquelle au moins un sous-ensemble desdits paramètres (U) est déterminé pendant une phase de calibration supervisée, semi-supervisée ou non supervisée, basée sur une pluralité d'images d'entrainement, les images d'entrainement comportant des yeux.

**4.** La méthode selon la revendication 3, dans laquelle lesdites images d'entrainement comprennent ou sont associées à des informations supplémentaires utilisées pour déduire la probabilité que l'utilisateur regarde un point donné.

**5.** La méthode selon l'une des revendications 1 à 4, utilisant une segmentation implicite ou explicite de l'image de l'œil de l'utilisateur en une pluralité de régions sémantiques ; de préférence, lesdites régions sémantiques comprenant une pluralité de régions parmi un groupe comprenant la cornée, la sclère et la peau ; de préférence, où la couleur ou le niveau d'échelle de gris observé dans chacune desdites régions sémantiques de l'œil dépend de ladite segmentation.

**6.** La méthode selon l'une des revendications 1 à 5, comprenant en outre :
b2) la rectification de ladite image comme si elle avait été vue depuis une posture de tête avec mise en forme canonique ;
de préférence, ladite étape de rectification comprenant l'ajustement d'un modèle facial en 3D spécifique à l'utilisateur avec ladite image.

**7.** La méthode selon l'une des revendications 1 à 6, ladite image comprenant au moins les yeux d'un utilisateur étant une image RGB-D, stéréoscopique, plénoptique ou en 3D.

**8.** La méthode selon l'une des revendications 1 à 7, ledit ensemble de paramètres (U) comprenant une pluralité de paramètres parmi :

le centre de rotation du globe oculaire (pc) ;
le décalage de l'axe visuel (k) ;
la distance du point nodal par rapport au pc (d) ;
le rayon du globe oculaire (re) ;
le rayon de la cornée (rc) ;
les coins de l'œil gauche et/ou droit (kl, kr).

**9.** La méthode de l'une des revendications 1 à 8, dans laquelle ladite étape pour déterminer l'axe visuel et/ou optique le plus probable comprend une détermination des ouvertures les plus probables des paupières supérieures et/ou inférieures.

**10.** La méthode selon l'une des revendications 1 à 9, dans laquelle ledit modèle d'observation est basé sur un modèle probabiliste ou statistique.

**11.** La méthode selon l'une des revendications 1 à 10, dans laquelle ledit ensemble de paramètres (U) définissant un modèle géométrique d'au moins un œil est rendu spécifique à l'utilisateur sur la base d'une pluralité d'images d'entraînement de l'œil dudit utilisateur.

**12.** La méthode de l'une des revendications 1 à 11, dans laquelle ladite étape pour déterminer l'axe visuel et/ou optique de l'œil (o, v) qui est le plus susceptible de produire lesdites données d'observation utilise des images incluant les deux yeux, et une contrainte sur la relation entre les mouvements des deux yeux.

**13.** La méthode selon l'une des revendications 1 à 12, dans laquelle ladite étape pour déterminer l'axe visuel et/ou optique (o, v) de l'œil dans une image implique une connaissance de cet axe visuel et/ou optique dans des images précédentes ou futures d'une même séquence.

**14.** Un appareil permettant d'estimer la direction du regard d'un utilisateur, comprenant les éléments suivants :

une caméra (22) pour capturer le visage d'un utilisateur ;
une base de données stockant des paramètres oculaires et faciaux spécifiques à l'utilisateur (21) ;

un système informatique (20) comprenant une mémoire sur laquelle est stocké un programme informatique comprenant un algorithme capable d'exécuter la méthode de l'une quelconque des revendications 1 à 13.

15. Un support de stockage lisible par ordinateur sur lequel est enregistré un programme informatique, le programme informatique comprenant un algorithme capable d'exécuter la méthode de l'une quelconque des revendications 1 à 13.

Fig. 1

```
┌─────────────────────────────────┐
│   obtaining observation data    │ ╱ b1
│   corresponding to an image of  │
│    at least one user's eye      │
└─────────────────────────────────┘
             │
             │  observation data X´, Λ
             ▼
┌─────────────────────────────────┐
│                                 │ ╱ b2
│      rectifying said image      │
│                                 │
└─────────────────────────────────┘
             │
             │  X,Λ,P_t
             ▼
┌─────────────────────────────────┐
│       generating user specific  │ ╱ b3
│    model at various orientations│
│  (o) and various eyelids operings (ue,le) │
└─────────────────────────────────┘
             │
   b  ↗      ▼
┌─────────────────────────────────┐
│                                 │ ╱ b4
│         inferring a visual      │
│    and/or optical axis of the eye │
└─────────────────────────────────┘
             │
             │  O
             ▼
┌─────────────────────────────────┐
│                                 │ ╱ b5
│    retrieving the gaze direction│
│                                 │
└─────────────────────────────────┘
```

Fig. 2

17

b22 → retrieving depth information

retrieving user's 3D facial model ← b22

$X$

$5$

fitting model and depth information — b23

determining head poseangle and translation — b24

$P_t = (R_t, T_t)$

b2

rectifying image to produce a front view warped image — b25

cropping portion of image including the eyes — b26

Fig. 3

Fig. 4a

Fig. 4b

Fig. 5

Fig. 6

Fig. 7

Fig. 8

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- A geometric approach to remote eye tracking. **A. VILLANUEVA.** Universal Access in the Information Society. Springer, 2009
- **D. WITZNER et al.** In the Eye of the Beholder: A survey of Models for Eyes and Gaze. *IEEE Transactions on pattern analysis and machine intelligence,* 2010, vol. 32 (3

- **A. VILLANUEVA.** A Novel Gaze Estimation System With One Calibration Point. *IEEE Transactions on systems, man and cybernetics-Part B,* 2008, vol. 38 (4